# EUROPEAN PATENT APPLICATION

(11) **EP 1 495 722 A1**
(43) Date of publication of application: **12.01.2005**
(21) Application number: 04014647.4
(22) Date of filing: 23.06.2004
(51) Int. Cl.: A61B 10/00

(54) **Ovulation cycle monitor system, toilet apparatus, and ovulation cycle monitor method**

(30) Priority: 09.07.2003 JP 2003272442
(71) Applicant: MATSUSHITA ELECTRIC INDUSTRIAL CO., LTD., Kadoma-shi, Osaka 571-8501 (JP)
(72) Inventor: Shiraishi,Takako, Ikoma-shi, Nara, 630-0121 (JP); Yamamoto, Hiroshi, Shijyonawate-shi, Osaka, 575-0013 (JP); Kaida, Manami, Nara-shi, Nara, 631-0021 (JP)
(74) Representative: Pautex Schneider, Nicole

(57) **Abstract**

An ovulation cycle monitor system 100 provided for a toilet fixture is composed of: a urine temperature measuring unit 102 that measures a temperature of urine excreted from a female user; a hormone measuring unit 103 that measures a concentration of a hormone that is contained in the excreted urine and fluctuates in its secretion amount with an ovulation cycle; a memory unit 117 that accumulates data of the measured urine temperature and data of the measured hormone concentration in association with a measurement day; an ovulation cycle calculating unit 104 that calculates an onset date and a length for each phase of the ovulation cycle by reference to the data of the measured urine temperature and the data of the measured hormone concentration accumulated in the memory unit; and an ovulation cycle predicting unit 115 that predicts future ovulation cycles of the female user on the basis of the onset date and the length calculated for each phase and directs a displaying unit 118 to display the predicted ovulation cycles.

## Description

### BACKGROUND OF THE INVENTION

### (1) Field of the Invention

The present invention relates to a measuring apparatus provided for a toilet fixture to measure a bodily fluid of a female user and, based on the measurement result, provide information regarding the ovulation (or menstrual) cycle including ovulation, menstruation, and a fertile phase. In particular, the present invention relates to an ovulation cycle monitor system and an ovulation cycle monitor method which support female health care and birth control.

### (2) Description of the Related Art

Various methods have been conventionally suggested to know the time of female ovulation as well as a fertile phase for practicing birth control.

In order for a woman to conceive, it is necessary to have intercourse during her fertile phase that lasts a few days starting a few days before ovulation and ending immediately after the ovulation. Then, in order to fertilize an ovum after the ovulation, it is essential to predict her ovulation date in advance. Conventionally, the basal body temperature method has been used in general to determine the ovulation date and fertile dates. With this method, she measures her basal body temperature and determines her ovulation or fertile dates on the basis that ovulation occurs while the basal body temperature shifts from a low temperature period to a high temperature period. However, in many cases where the basal body temperature method is used, the female user will know the end of her low temperature period only after her body temperature enters in the high temperature period (namely, her infertile phase). For this reason, it is difficult to predict her fertile phase in advance only through the measurement of her basal body temperature.

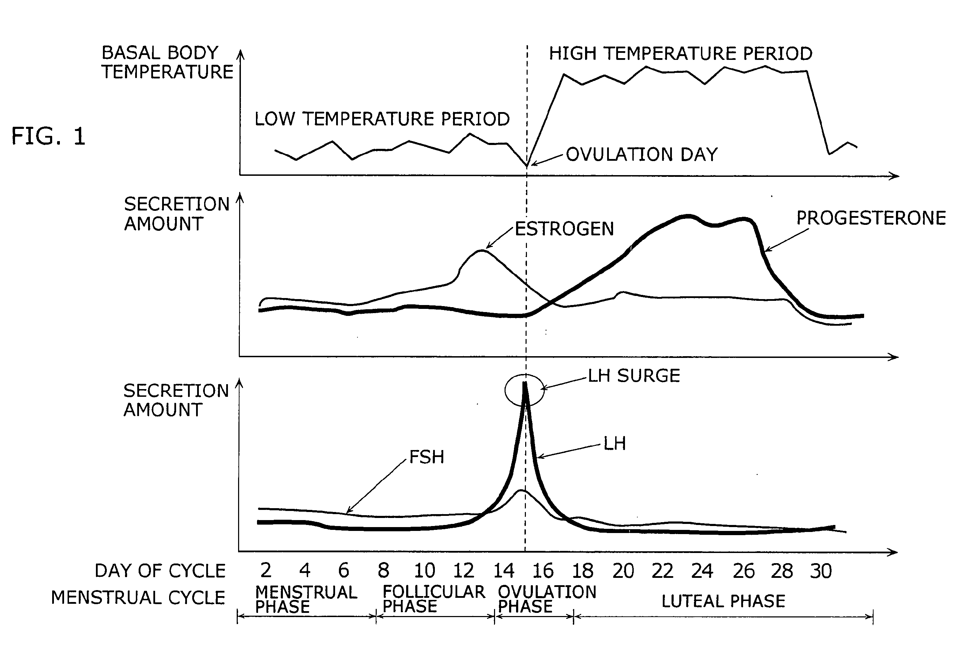

It is generally well known that ovulation occurs in association with the secretion of a certain kind of hormone that fluctuates in amount with the phases of the menstrual cycle. FIG. 1 is a graph showing the fluctuations in the basal body temperature and the fluctuations in the amounts of hormones secreted during the menstrual cycle. As the hormones related to the female menstrual cycle, there are estrogen (follicular hormone), progesterone (corpus luteum hormone), follicle-stimulating hormone (FSH), and luteinizing hormone (LH), as shown in FIG. 1. The amount of secretion varies for each hormone, depending on the phases of the menstrual cycle. Estrogen is produced mainly from ovarian theca cells, granulosa cells, and luteal cells, and it surges before ovulation. Progesterone is a typical corpus luteum hormone that is produced mainly in the corpus luteum of ovary, theca cells, zona reticularis of the adrenal cortical, and the placenta, and it surges after ovulation. Both estrogen and progesterone exhibit feedback influences upon a mechanism of hormone regulation made up of the hypothalamus and the pituitary gland, and act to adjust the production of FSH and LH. Both FSH and LH are secreted by basophil cells of the anterior lobe of the hypophysis, and their production and release are adjusted by a gonadotropin-releasing hormone secreted from the hypothalamus. LH acts upon the ovarian theca cells and stimulates the production of androgen. Also, LH acts upon the granulosa cells of a mature follicle to cause ovulation and luteinization, and stimulates the hormone secretion from the corpus luteum. Meanwhile, FSH acts upon the granulosa cells to stimulate the development of the ovarian follicle. In addition, FSH converts androgen to estrogen supplied by the theca cells, as well as guiding the luteinizing hormone receptors of the granulosa cells.

At the beginning of the follicular phase, the ovarian follicle is immature and the estrogen level is low, meaning that the controlling action on FSH secretion by estrogen is weak. This leads to a rise in the FSH level and, as a result, the development of the ovarian follicle is stimulated. From the middle to late in the follicular phase, by the increase of estrogen due to the maturation of the ovarian follicle, the FSH level is controlled to decline. In the ovulation phase, the level of estrogen drastically increases, exhibiting a positive feedback influence upon the mechanism of hormone regulation made up of the hypothalamus and the pituitary gland. This results in the drastic increase of LH (called "LH surge"). Following the LH surge, ovulation occurs within 24 to 36 hours. In the luteal phase, the rising level of progesterone exhibits a negative feedback influence upon the mechanism of hormone regulation, so that the levels of FSH and LH drop. Late in the luteal phase, the levels of estrogen and progesterone decrease, meaning that the level of FSH is released from the negative feedback and accordingly increases. The explanation given so far is described in the non-patent document, "Sankafujinkagaku (Obsterics and gynecology)" supervised by Koichi Kato, published by Herusu Shuppan in 1999 (pages 13-20).

Based on the biological structure as explained above, a technology has been conventionally suggested. To be more specific, with attention being focused on biological hormones that vary in their levels in association with the ovulation cycle, data of the basal body temperature is monitored and the urinary hormones that vary in their levels depending on the phases of the ovulation cycle are measured on an as needed basis. Using the measurement result, the ovulation cycle that is predicted based on the basal body temperature may be corrected, or the accuracy of such prediction may be increased. Thus, the timing of measuring the urinary hormones is specified, so that the measurement can be efficiently achieved. This conventional technology is disclosed in Patent Publication No. 3201412, for example.

With the conventional technology, a more accurate determination of the ovulation cycle is possible because both the urinary hormones and the basal body temperature are measured. However, it should be noted that they have to be measured each day. A female basal body temperature is measured using a basal thermometer each morning before she gets out of bed. She has to lie quietly in bed for a few minutes during the measurement otherwise a movement will give an inaccurate temperature reading. After this, on the other hand, she has to go to the bathroom to collect her urine then measures the urinary hormones. In this way, the basal body temperature and urinary hormones have to be measured at the separate locations, i.e., bedroom and bathroom, and this requires time and effort. Also, when waking up in the morning, she may inadvertently get up before measuring the basal body temperature, so that the accurate basal body temperature cannot be obtained. Or, she may forget to take the basal body temperature itself. Furthermore, it has been reported that a mercury oral thermometer was broken while using and mercury spilled inside the user's mouth. This accident happened because the user carelessly fell asleep again after placing the thermometer under her tongue. As is often the case, one wakes up early in the morning, feeling the urge to go to the bathroom. Nonetheless, she has to resist the urge and wait for a few minutes while she is taking her basal body temperature. As a consequence of this inconvenience, although the basal body temperature needs to be continuously measured for a period of time as long as at least three cycles to obtain the basal body temperature data, many women give up in frustration because it is troublesome to keep measuring the basal body temperature.

### SUMMARY OF THE INVENTION

The present invention has been conceived in view of the above problem, and it is an object of this invention to provide an ovulation cycle monitor system that saves a user from having to measure the basal body temperature and allows the user to accurately determine the user's ovulation cycle.

To achieve the stated object, the ovulation cycle monitor system of the present invention that is provided for a toilet fixture is made up of: a urine temperature measuring unit operable to measure a temperature of urine excreted from a female user; a hormone concentration measuring unit operable to measure a concentration of a hormone that is contained in the excreted urine and fluctuates in a secretion amount with an ovulation cycle; a memory unit operable to accumulate data of the measured urine temperature and data of the measured hormone concentration in association with a measurement day; and a determining unit operable to determine the ovulation cycle of the female user by reference to the fluctuations in the urine temperature and in the hormone concentration accumulated in the memory unit. Accordingly, the ovulation cycle monitor system of the present invention allows the user to determine the ovulation cycle with a higher degree of accuracy on the basis of the urine temperature and hormone concentrations measured through usual urination without having to bother about taking the user's basal body temperature. The ovulation cycle monitor system according to Claim 1,

Moreover, the determining unit includes: an ovulation cycle calculating unit operable to calculate an onset date and a length for each phase of the ovulation cycle by reference to the data of the measured urine temperature and the data of the measured hormone concentration accumulated in the memory unit; and an ovulation cycle determining unit operable to determine future ovulation cycles of the female user on the basis of the onset date and the length calculated for each phase.

Furthermore, the ovulation cycle monitor system includes an indicating unit operable to indicate the determined ovulation cycle to the female user, wherein the indicating unit indicates at least one of a menstrual phase, an ovulation phase, a follicular phase, a luteal phase, and a fertile phase which make up the ovulation cycle of the female user.

It should be noted here that the present invention may be realized not only as the above-stated ovulation cycle monitor system, but as: an ovulation cycle monitor method having steps corresponding to the characteristic units provided in the ovulation cycle monitor system; and a program that causes a computer to execute these steps. Also, it should be understood that such a program can be distributed via a record medium such as a CD-ROM as well as via a transmission medium such as the Internet.

Accordingly to the present invention, instead of measuring the basal body temperature, the ovulation cycle monitor system measures the female user's urine temperature and urinary hormone concentrations from her urine normally excreted, and determines her ovulation cycle on the basis of the fluctuations in the measured urine temperature and in the measured hormone concentrations. Therefore, the female user is freed from the burden of measuring her basal body temperature each morning when she wakes up. In addition, the ovulation cycle is predicted on the basis of both the urine temperature and the urinary concentrations of hormones which fluctuate in their secretion amounts with the phases of the ovulation cycle. On account of this, the present system can predict the ovulation cycle with a higher degree of accuracy and provide information with a high degree of reliability that can be used by the user when she plans her activity schedule.

Moreover, the ovulation cycle predicting unit predicts each phase of the ovulation cycle including the menstrual phase, ovulation phase, etc. according to the calculated length of the corresponding phase. This means that the ovulation cycle monitor system can provide the user with the user's ovulation date, date of menstruation onset, etc. of her next ovulation cycle viewed from the current day, as the prediction result. Therefore, it becomes convenient to use such information for both a woman who wishes to practice contraception and a woman who wishes to become pregnant when they plan their activity schedules including trips and intercourses in advance. Especially for a woman who has an unpleasant symptom like dysmenorrheal and premenstrual syndrome peculiar to a certain phase of the ovulation cycle, the system of the present invention is useful because she can plan her daily life in advance to avoid a hard schedule to relax during the certain phase.

Also, according to the ovulation cycle monitor system of the present invention, the user's urinary hormone concentrations are continuously measured for a long period of time. In consequence, the system can provide information that is useful for early detection of hormone imbalance or diseases peculiar to women.

Moreover, using the ovulation cycle monitor system of the present invention, the user can grasp her menstrual cycle, ovulation cycle, and hormone balance. On account of this, the present system can be a useful tool for a woman who is on the pill or under hormone treatment. Specifically, for the dosing management and health care, she can check the measurement results of her urine temperature and hormone secretions for herself and can also show them to a specialist, such as her physician.

### BRIEF DESCRIPTION OF THE DRAWINGS

These and other objects, advantages and features of the invention will become apparent from the following description thereof taken in conjunction with the accompanying drawings that illustrate a specific embodiment of the invention. In the Drawings:
FIG. 1 is a graph showing fluctuations in the basal body temperature during the menstrual cycle and fluctuations in the secretion amounts of hormones;
FIG. 2 is a functional block diagram showing the construction of an ovulation cycle monitor system of an embodiment of the present invention;
FIG. 3 shows an example of an external view of a toilet apparatus realizing the ovulation cycle monitor system shown in FIG. 2;
FIG. 4 is a flowchart showing an operation performed by units provided for the ovulation cycle monitor system when a user uses the toilet apparatus shown in FIG. 3;
FIG. 5 shows an example of measurement data accumulated in a memory unit shown in FIG. 2;
FIG. 6 is a flowchart showing an operation performed by an ovulation cycle predicting unit shown in FIG. 2 from predicting an ovulation cycle of a user to indicating the predicted ovulation cycle to the user;
FIG. 7 shows an example of a calendar that is displayed on a liquid crystal display shown in FIG. 3; and
FIG. 8 shows an example of an e-mail screen sent to notify a previously registered partner of the onset of the user's fertile phase.

### DESCRIPTION OF THE PREFERRED EMBODIMENT

The following is an explanation of an embodiment according to the present invention, with reference to the drawings.

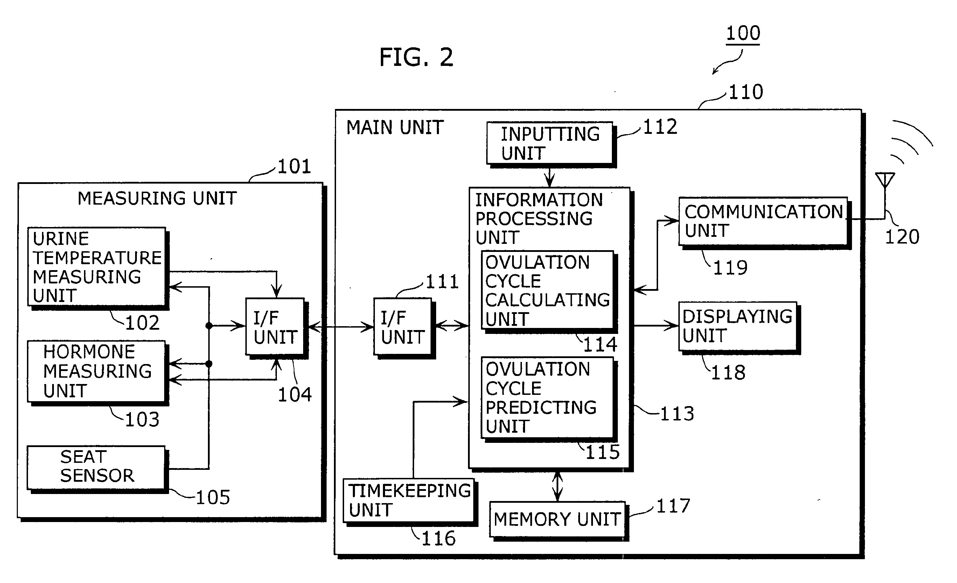

FIG. 2 is a functional block diagram showing the construction of an ovulation cycle monitor system 100 of the embodiment of the present invention. The ovulation cycle monitor system 100 is set inside a toilet fixture, and measures the urine temperature and the urinary hormone concentrations of a female user to monitor a cycle related to ovulation. The ovulation cycle monitor system 100 is composed of a measuring unit 101 and a main unit 110 which are mutually connected via short-range radio or a cable (external bus). The measuring unit 101 is mounted inside a toilet bowl and provided with a urine temperature measuring unit 102, a hormone measuring unit 103, an I/F unit 104, and a seat sensor 105. The urine temperature measuring unit 102 measures the temperature of urine right after excretion using an infrared sensor or the like. The hormone measuring unit 103 is provided with a urine collecting unit (not shown) that collects the user's urine into a urine receiver and measures the hormone concentrations in the collected urine. More specifically, a test reagent that varies in color or turbidity when specifically reacting with a specific hormone is used. A urinary hormone concentration is obtained by optically measuring a concentration of mixed liquid of urine and test reagent using a spectrophotometer or the like. In the present embodiment, the hormone measuring unit 103 starts measuring the hormone concentrations three days before a predicted date of the LH surge according to a control signal inputted from the main unit 110, and stops the measurement after obtaining the peak of the LH surge. The I/F unit 104 converts data of the urine temperature measured by the urine temperature measuring unit 102 and data of the hormone concentrations measured by the hormone measuring unit 103 into a transmission format, then sends these sets of data to the main unit 110 via the short-range radio or cable. The I/F unit 104 also sends the control signal that has been sent from the main unit 110 to the hormone measuring unit 103. The seat sensor 105 is a pressure sensor or the like that is set between a toilet seat and the toilet bowl to detect a weight when the user sits on the toilet seat. When detecting the user sitting down, the seat sensor 105 outputs a sitting detection signal to the urine temperature measuring unit 102, the hormone measuring unit 103, and the I/F unit 104 so that each processing unit of both the measuring unit 101 and the main unit 110 may be set on standby. To be more specific, the sitting detection signal inputted into the I/F unit 104 is sent to the main unit 110 via the short-range radio or cable so that each processing unit of the main unit 110 may be also set on standby.

The main unit 110 is mounted on a bathroom wall, for example, and is connected with the measuring unit 101 via the short-range radio or cable. The main unit 110 is provided with an I/F unit 111, an inputting unit 112, an information processing unit 113, a timekeeping unit 116, a memory unit 117, a displaying unit 118, a communication unit 119, and an antenna 120. The I/F unit 111 converts data received from the measuring unit 101 via the short-range radio or cable into a format that can be processed in the main unit 110, then outputs the resultant converted data to the information processing unit 113. The inputting unit 112 receives inputs through an operation panel including a keypad to be manipulated by the user. The input examples are: identifying information (nickname or name, for example) to identify an individual user; information regarding the user's body (the user's age, height, and weight, or whether the user has reached menopause, for example); an e-mail address to which a notification about the onset of the user's fertile phase is sent; and a password for the user to obtain an ovulation cycle calendar from a personal digital assistant (PDA). The information processing unit 113 calculates the user's ovulation cycle from the urine temperature and the urinary hormone concentrations measured by the measuring unit 101, and is provided with an ovulation cycle calculating unit 114 and an ovulation cycle predicting unit 115.

The ovulation cycle calculating unit 114 accumulates the urine temperature and the urinary hormone concentrations both sent from the measuring unit 101 into the memory unit 117 for each user. By reference to the accumulated urine temperature and urinary hormone concentrations, the ovulation cycle calculating unit 114 calculates a length for each phase of the user's ovulation cycle, that is, the menstrual phase, the ovulation phase, the fertile phase, etc. Since urine is retained in the deep-lying bladder and is less subject to the outside air temperature, the urine temperature is close to the deep body temperature. In particular, the first morning urine well reflects the deep body temperature at rest during sleep. Thus, as is the case with the basal body temperature, on the basis of the first morning urine, fluctuations in the body temperature that is influenced by the hormones during the ovulation cycle can be monitored. To be more specific, when receiving the measurement result from the measuring unit 101, the ovulation cycle calculating unit 114 creates a message so that the user may input the identifying information, such as her name or nickname, and directs the displaying unit 118 to display this message. Based on the inputted identifying information, the ovulation cycle calculating unit 114 determines the user to whom the measurement result including the urine temperature and the urinary hormone concentrations belongs, and accumulates the result in association with the user into the memory unit 117. In doing so, the ovulation cycle calculating unit 114 refers to a table previously stored in the memory unit 117, for example, and classifies the measured hormone concentrations according to four levels indicated as "-", "+", "++", and "+++." After this classification, the ovulation cycle calculating unit 114 accumulates each hormone concentration represented as the classified level. In addition, the ovulation cycle calculating unit 114 obtains a date and time, at which the measurement result was received from the measuring unit 101, from the timekeeping unit 116, and accumulates the date and time together with the measurement result into the memory unit 117.

On the basis of the ovulation cycle calculated by the ovulation cycle calculating unit 114, the ovulation cycle predicting unit 115 predicts a future ovulation cycle and creates a message to be displayed by the displaying unit 118 as well as an e-mail to be sent to a pre-registered destination address. Moreover, according to a data readout command received by the communication unit 119, the ovulation cycle predicting unit 115 reads information, such as a calendar showing the ovulation cycle, from the memory unit 117, and then outputs the read information to the communication unit 119. In addition to the clock function for timekeeping the current time of day, the timekeeping unit 116 is provided with a calendar function for timekeeping the current year, month, and day. The memory unit 117 stores: a table showing a threshold etc. used for classifying the urinary hormone concentrations according to the stated four levels; a table showing determination standards used for determining each phase of the ovulation cycle from the fluctuations in the urine temperature and those in the urinary hormone concentrations; a file including character strings and images which are to be used in creating a message, calendar, etc.; and a setting which is previously inputted by the user. The memory unit 117 accumulates the urine temperature and the urinary hormone concentrations measured by the measuring unit 101 for each user, and has a storage area to store a list of users, a most recently formed calendar, etc. The displaying unit 118 displays information, such as a message or a user's ovulation cycle calendar created by the information processing unit 113. Following an instruction by the ovulation cycle predicting unit 115, the communication unit 119 sends an e-mail notifying a user's partner of the onset of the user's fertile phase which has been predicted by the ovulation cycle predicting unit 115, via a cellular phone communication network or the like, to an address (of a cellular phone used by the partner, for example) that is pre-registered in association with the user. The communication unit 119 also receives a command sent from a user's cellular phone etc. and sends corresponding data such as an ovulation cycle calendar in response to the received command. The antenna 120 receives/sends radio waves, such as a cellular phone communication network.

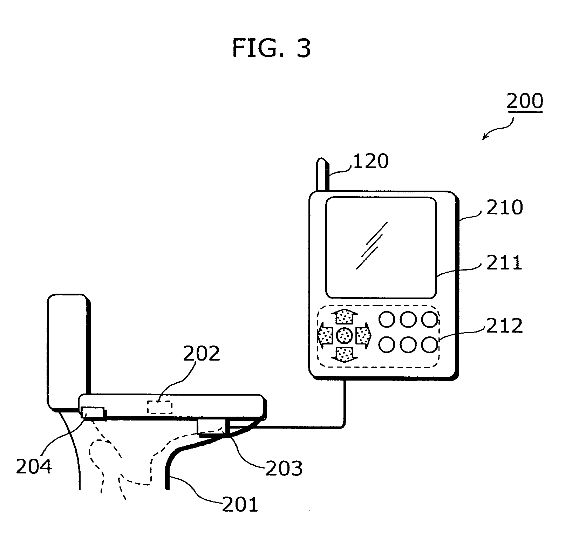

FIG. 3 shows an example of an external view of a toilet apparatus 200 realizing the ovulation cycle monitor system 100 shown in FIG. 2. As shown in FIG. 3, the toilet apparatus 200 is composed of: a toilet bowl 201 inside which the measuring unit 101 shown in FIG. 2 is mounted; and a main unit 210 which processes the information obtained by the measuring unit 101 and directs the displaying unit 118 to display the processing result to the user. The main unit 210 shown in FIG. 3 is an example of the main unit 110 shown in FIG. 2. As shown in FIG. 3, the toilet bowl 201 of the toilet apparatus 200 is provided with an infrared sensor 202, a hormone measuring unit 203, and a seat sensor 204. The infrared sensor 202, which is an example of the urine temperature measuring unit 102 shown in FIG. 2, detects infrared radiation to measure the temperature of the excreted urine. The hormone measuring unit 203, which is an example of the hormone measuring unit 103 shown in FIG. 2, collects the excreted urine into the urine receiver (not shown) and mixes the collected urine with a test reagent to optically measure a urinary hormone concentration. The seat sensor 204, which refers to the seat sensor 105 shown in FIG. 2, detects a weight when the user sits on the toilet seat and outputs a sitting detection signal to set each unit of both the measuring unit 101 and the main unit 110 on standby.

The main unit 210 of the toilet apparatus 200 is provided integrally with a liquid crystal display 211, an operation panel 212 including a keypad, and the antenna 120. The liquid crystal display 211 is an example serving as both the displaying unit 118 and the inputting unit 112, and a tablet and the like are laminated on its lower layer. The liquid crystal display 211 displays a calendar showing the user's ovulation cycle as well as a message requesting the user to input the identifying information and the body information. Also, the liquid crystal display 211 displays input buttons and, when an area of a displayed button is pressed, inputs a signal corresponding to a coordinate position of that button from the tablet of the lower layer. The operation panel 212 is an input manipulating unit used by the user to switch the contents displayed on the liquid crystal display 211 and to input the user's identifying information and body information. The antenna 120 receives/sends radio waves of radio communication network, such as a cellular phone communication network.

Next, with reference to FIGS. 3 to 7, an explanation is given for an operation performed by the units provided for the ovulation cycle monitor system 100 when the user uses the system 100 having the construction as described so far.

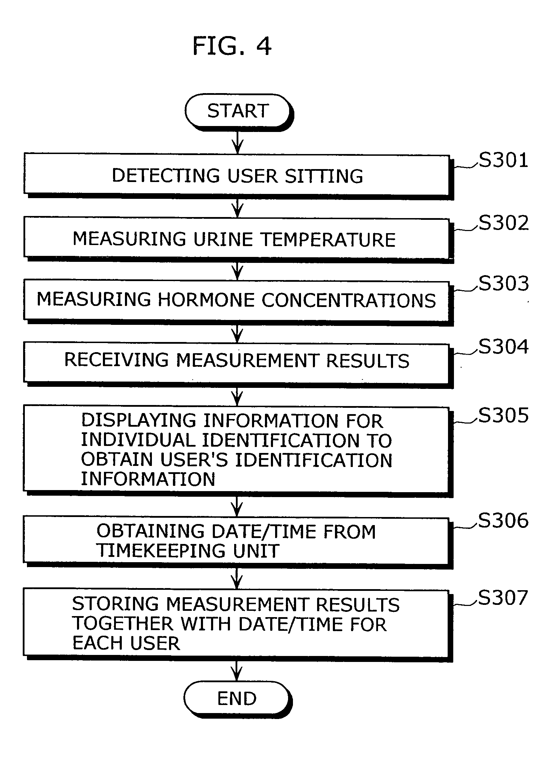

FIG. 4 is a flowchart showing an operation performed by the units provided for the ovulation cycle monitor system 200 when the user uses the toilet apparatus 200 shown in FIG. 3. When the female user sits on the toilet seat, the seat sensor 204 detects the user sitting down (S301) and outputs the sitting detection signal to each unit of both the measuring unit 101 and the main unit 110. With this signal, the infrared sensor 202 is set into a standby state and measures the temperature of urine passing across a temperature detection range (S302). At the same time, receiving the sitting detection signal via the I/F unit 104 and the I/F unit 111, the ovulation cycle predicting unit 115 outputs a control signal to direct the hormone measuring unit 203 to measure the urinary hormone concentrations. Here, the unit 115 outputs this signal three days before the date of the LH surge, on the basis of the current date/ time and the prediction result given by the ovulation cycle calculating unit 114. If the LH surge is not detected even after the predicted date of the LH surge (in other words, the sharp rise and subsequent fall of the LH concentration is not seen), the ovulation cycle predicting unit 115 outputs a control signal to direct the hormone measuring unit 203 to keep measuring the urinary hormone concentrations until the LH surge, an increase of progesterone, or a rise in the urine temperature is detected. At all other times except when the LH surge is not detected, the ovulation cycle predicting unit 115 does not output the control signal directing the hormone measuring unit 203 to measure the urinary hormone concentrations.

Receiving the control signal from the ovulation cycle predicting unit 115 via the I/F unit 104 and the I/F unit 111, the hormone measuring unit 203 has the urine receiver operate in synchronization with the user sitting and collect the excreted urine. The hormone measuring unit 203 divides the collected urine by the number of hormones to be measured and mixes a test reagent for each divided portion of urine so as to measure the concentrations of hormones, such as LH, progesterone, estrogen, and FSH (S303). After the end of the measurements of urine temperature and hormone concentrations, each measurement result is sent to the information processing unit 113 of the main unit 210 via the I/F unit 104 and the I/F unit 111 (S304). The ovulation cycle calculating unit 114 of the information processing unit 113 invokes the list of users that is previously stored in the memory unit 117, and directs the liquid crystal display 211 to list the identifying information, such as the users' names or nicknames. It should be noted here that the identifying information, such as a user's ID, name, age, height, weight, and nickname, is previously stored in the memory unit 117 for each user in the present embodiment. The user verifies the list of identifying information, namely a list of nicknames for example, displayed on the liquid crystal display 211 and selects her identifying information by manipulating the operation panel 212 following a prompting guidance (S305). Note that if there are no inputs from the user after a set period of time has passed after the identifying information list and prompting guidance were displayed, for example, the ovulation cycle calculating unit 114 categorizes the received result as the measurements belonging to a specific individual and stores them into the memory unit 117. The ovulation cycle calculating unit 114 obtains the date and time, at which the measurement result was received, from the timekeeping unit 116 (S306). Then, the unit 114 stores the obtained date and time together with the received measurements into the memory unit 117. When doing so, the ovulation cycle calculating unit 114 identifies the user based on the selected identifying information, and stores the measurements and their received date/time together with the profile information such as the body information of the identified user, as the user's individual measurement data (S307). Then, the processing is terminated here.

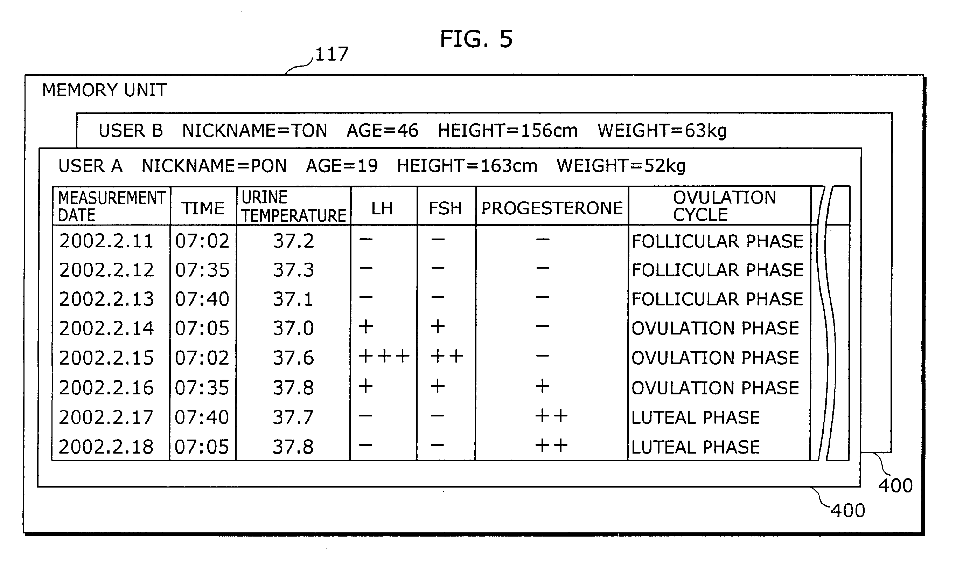

FIG. 5 shows an example of measurement data 400 accumulated in the memory unit 117 shown in FIG. 2. Note that the measurement results of estrogen are omitted in this example. As shown in FIG. 5, a user ID (such as "user A" or "user B") used by the information processing unit 113 to identify the user and a pre-registered nickname (such as "Pon" or "Ton") used by the user to distinguish herself from others are affixed to the measurement data 400 that is accumulated for each user in the memory unit 117. Moreover, the body information of the user (such as age, height, and weight) that has been previously registered by the user is also affixed to the measurement data 400. In each set of the measurement data 400, there are columns for: the measurement date; time at which the measurement result is received; urine temperature, LH concentration, FSH concentration, progesterone concentration which are all measurement results given by the measuring unit 101; and ovulation cycle which is a prediction result given by the ovulation cycle calculating unit 114. Here, each hormone concentration in the columns is classified by the ovulation cycle calculating unit 114 and expressed as one of the four levels. At the point in time when the urine temperature and urinary hormone concentrations are only measured, the column for the ovulation cycle is blank. After the user manipulates the operation panel 212 to display the ovulation cycle on the liquid crystal display 211 and the ovulation cycle calculating unit 114 reaches a determination, the determination result is written in the blank. As shown in FIG. 5, the column of the ovulation cycle shows the corresponding phase in the ovulation cycle to which the measurement date belongs, as the determination result given by the ovulation cycle calculating unit 114.

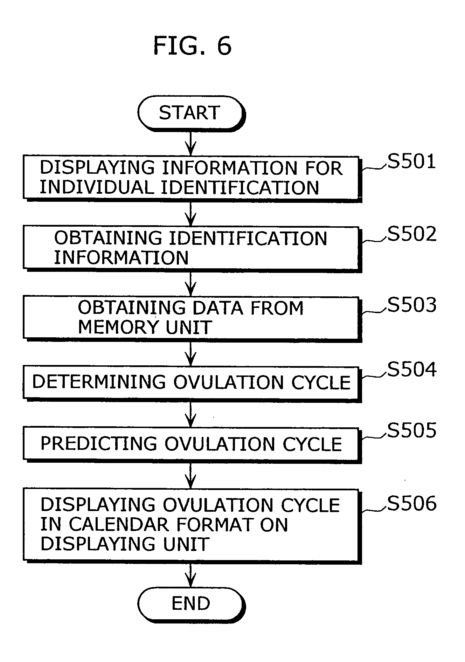

Next, with reference to FIG. 6, an explanation is given for an operation performed by the ovulation cycle predicting unit 115 of the ovulation cycle monitor system 100 shown in FIG. 2. FIG. 6 is a flowchart showing the operation performed by the ovulation cycle predicting unit 115 from predicting the user's ovulation cycle to indicating the predicted ovulation cycle to the user.

First, when the user would like to know which phase of the ovulation cycle she is currently in, the user pushes a start button (not shown) of the operation panel 212 provided for the main unit 210. In response to this, the ovulation cycle calculating unit 114 directs the liquid crystal display 211 to display a user identification screen in order for the user to select herself among the others previously registered into the memory unit 117. On the user identification screen, the users' nicknames, for example, are listed and the user can input her identifying information by selecting her pre-registered nickname (S501). When the user selects her nickname ("Pon" shown in FIG. 5, for example), the ovulation cycle calculating unit 114 obtains the corresponding identifying information of the user (data of "user A" shown in FIG. 5, for example) (S502). On the basis of the obtained identifying information, the ovulation calculating unit 114 specifies the corresponding measurement data 400 individually stored in the memory unit 117, and obtains accumulation data made up of the urinary hormone concentrations and urine temperature from the measurement data 400 (S503). With reference to the table stored in the memory unit 117, the ovulation cycle calculating unit 114 calculates the user's ovulation cycle on the basis of the fluctuations in the urinary hormone concentrations and in the urine temperature accumulated in the individual measurement data 400. Moreover, in accordance with the calculated ovulation cycle, the ovulation cycle calculating unit 114 determines the phase which the user is currently in (S504).

A more detailed explanation is given. As shown in FIG. 1, the graph showing the basal body temperature can be divided into two parts. That is, the basal body temperature is usually low from the menstrual phase to the ovulation phase (referred to as the low temperature period) and usually high during the luteal phase that lasts from ovulation to the onset of the next menstrual phase (referred to as the high temperature period). As explained above, estrogen sharply increases before ovulation while progesterone sharply increases after ovulation. At the beginning of the follicular phase, the estrogen level is low, meaning that the controlling action on FSH secretion by estrogen is weak. This leads to a rise in the FSH level. From the middle to late in the follicular phase, the FSH level is controlled to decline due to the increase of estrogen. In the ovulation phase, the level of estrogen drastically increases, resulting in the drastic increase of LH (called "LH surge"). Following the LH surge, ovulation occurs within 24 to 36 hours. Late in the luteal phase, the levels of estrogen and progesterone decrease and, thus, the level of FSH rises. Based on these fluctuations in the secretion amounts of hormones and in the urine temperature during the ovulation cycle, the ovulation cycle calculating unit 114 invokes the past measurement results of the urinary hormone concentrations and the urine temperature that are individually stored in the memory unit 117 and performs calculation. Then, the ovulation cycle calculating unit 114 determines the phase which the user is currently in, i.e., the menstrual, follicular, ovulation, or luteal phase. The unit 114 gives the determination result on a timescale measured in days.

The ovulation cycle calculating unit 114 determines the user's ovulation cycle in accordance with the combination of the urine temperature and the secretion amounts of hormones (namely, LH, FSH, progesterone, and estrogen). The body temperature varies from individual to individual. For this reason, the ovulation cycle calculating unit 114 divides the urine temperature data into two parts by judging the temperature is within her high or low temperature period for each user. When doing so, the unit 114 considers her average, highest, and lowest temperatures obtained from her urine temperature data over the past month or a longer duration. Moreover, the ovulation cycle calculating unit 114 classifies the secretion amounts of hormones into four levels (i.e., "-", "+", "++", and "+++") ranging from the lowest level to the highest level during the ovulation cycle, according to the standard fluctuations observed for each phase as shown in FIG. 1. The unit 114 makes a determination from a combination pattern of the user's body temperature and the measured secretion amounts of hormones. For instance, suppose the combination pattern shows that: the urine temperature is classified as in the low temperature period; the LH level is as "+" meaning low; the FSH level is as "+" meaning low; and the progesterone level is as "-" meaning the lowest. In this case, the user is judged to be currently in the follicular phase before ovulation. As another instance, suppose the combination pattern shows that: the urine temperature is classified as in the low temperature period; the LH level is as "++" meaning high, or "+++" meaning the highest; and the FSH level is as "++" meaning high. In this case, the user is judged to be currently in the ovulation phase during which she becomes most fertile. On the other hand, suppose the pattern shows that: the urine temperature is classified as in the high temperature period; the LH level is as "-" meaning the lowest; the FSH level is as "-" meaning the lowest; and the progesterone level is as "++" meaning high, or "+++" meaning the highest. In this case, the user is judged to be currently in the luteal phase during which she becomes infertile.

Back to the flowchart in FIG. 6, the ovulation cycle predicting unit 115 finds the ovulation cycle pattern of the user using the past data accumulated in the measurement data 400 and predicts the user's ovulation cycle from the current day onward (S505). The high temperature period after ovulation (namely, the luteal phase), that lasts from ovulation to the onset of the next menstruation, does not vary among individuals so much, and its length is constantly about 14 days with a discrepancy of two days. The ovulation cycle predicting unit 115 predicts the user's next ovulation cycle, according to the fact that the high temperature period after ovulation lasts approximately 14 days for most women and the average length that is obtained from the past data for each phase, a period from the onset of menstruation to the LH surge, and the like. Note here that, on the day of the onset of menstruation, the urine temperature shifts from the high temperature period to the low temperature period and also the secretion amount of estrogen drops to the lowest level (i.e., "-"). The user may be guided to input the date of the onset of her last menstruation from the inputting unit 112, so that the menstrual phase of the predicted ovulation cycle will be corrected. In this case, the ovulation cycle calculating unit 114 sets the inputted date of the onset of her menstruation as the first day of her ovulation cycle and also corrects the onset date for each phase.

Meanwhile, the information processing unit 113 has a calendar displaying function and directs the liquid crystal display 211 to display all the phases of the user's ovulation cycle in calendar format on the basis of the prediction result given by the ovulation cycle calculating unit 114 or the ovulation cycle predicting unit 115. More specifically, the liquid crystal display 211 indicates the ovulation, menstrual, fertile, and infertile (i.e., the follicular phase after ovulation) phases using respective marks on the displayed calendar (S506).

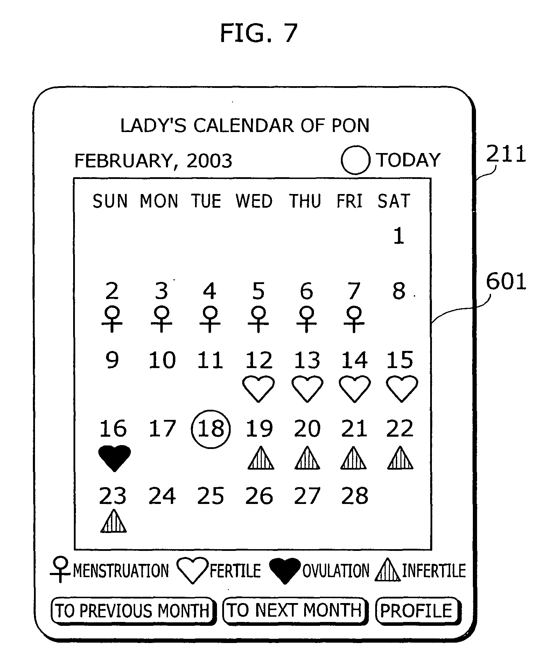

FIG. 7 shows an example of a calendar 601 that is displayed on the liquid crystal display 211 shown in FIG. 3. In this example of FIG. 7, the calendar 601 of February in the year 2003 that belongs to the user whose nickname is "Pon" is shown. On the calendar 601, the Pon's menstrual, fertile, ovulation, and infertile days are indicated with respective marks. To be more specific, her menstrual phase lasts from the 2^{nd} to the 7^{th}, her fertile phase lasts from the 12^{th} to the 15^{th}, her ovulation day is the 16^{th}, and her infertile phase lasts from the 19^{th} to the 23^{rd}. As shown on the calendar 601, the current date is encircled by the mark "O", and it is "the 18^{th} of February in 2003" in this example. Thus, each phase of her ovulation cycle before the 18^{th} of February (i.e., the menstrual, fertile, and ovulation phases) is indicated on the basis of the determination result given by the ovulation cycle calculating unit 114. Meanwhile, the infertile phase of her ovulation cycle following the 18^{th} is indicated on the basis of the prediction result given by the ovulation cycle predicting unit 115. At the lower part of the screen of the liquid crystal display 211, display buttons such as "TO PREVIOUS MONTH," "TO NEXT MONTH," and "PROFILE" are displayed. By pressing one of these display buttons, the user can have the liquid crystal display 211 show the previous or next month calendar, or information regarding the user's body (age, height, and weight). It should be noted here that such prediction result may be indicated by a graph showing the fluctuations in the urinary hormone concentrations and in the urine temperature. Also, each phase may be indicated by dates, and its length may be indicated by the number of days. Alternatively, dates may be displayed in a different color for each phase of the ovulation cycle on the calendar.

Moreover, the ovulation cycle calculating unit 114 detects hormone imbalance with reference to the table stored in the memory unit 117 and directs the liquid crystal display 211 to display so. For example, when a difference between the highest and lowest levels of a hormone concentration is less than a standard level shown in the table, the ovulation cycle calculating unit 114 judges whether the user's hormone is in or off balance by comparing the user's hormone concentrations at the highest and lowest levels to the respective standards. If judging that the user's hormone is off balance, the ovulation cycle calculating unit 114 directs the displaying unit 118 to display a message suggesting for the user to see her physician or gynecologist if the user has an unpleasant symptom.

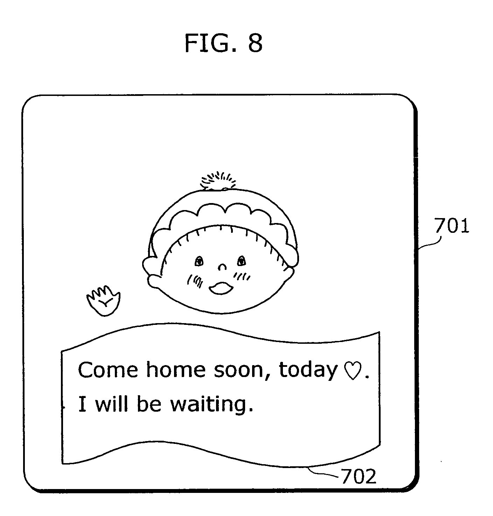

In addition to indicating the ovulation cycle to the user using the liquid crystal display 211, the ovulation cycle predicting unit 115 of the information processing unit 113 is provided with a function with which the onset of the user's fertile phase is sent by e-mail via the communication unit 119 to the address previously registered by the user. FIG. 8 shows an example of an e-mail screen 701 sent to notify the pre-registered partner of the onset of the user's fertile phase. On the e-mail screen 701, a message 702 saying "Come home soon, today 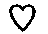 . I will be waiting." inputted by the user in advance is displayed together with an image of a baby. The baby image on the e-mail screen 701 was selected by the user from a plurality of images previously stored in the memory unit 117, for example. In a case where the user previously sets to notify the partner of the onset of her fertile phase by e-mail, the information processing unit 113 reads out a notification mail as shown in FIG. 8 from the memory unit 117 at the time of the predicted onset of her fertile phase and sends it via the communication unit 119 to the pre-registered mail address of the cellular phone, personal computer, or the like used by the user's partner.

Accordingly, the user can use the ovulation cycle monitor system 100 of the present embodiment for birth control including health care, contraception, and fertilization by knowing the phase of the ovulation cycle which she currently is in. For example, for a woman who is suffering from an unpleasant symptom caused by premenstrual syndrome (PMS) which remarkably emerges during the follicular phase, her uneasiness and impatience can be lessened by knowing that she is in the phase of feeling this way. Moreover, she can ease the symptom by trying something for a refreshing change of pace or trying to lead a slow life without frustrations. Meanwhile, a woman who does not wish to become pregnant will pay more attention to birth control during her ovulation phase at which she is most fertile. On the other hand, a woman who wishes to become pregnant can increase her chance of pregnancy by having intercourse during her ovulation phase. In addition, the chance of pregnancy can be further increased because the onset of the user's fertile phase is automatically notified not only to the user herself but also to her partner.

According to the ovulation cycle monitor system 100 of the present embodiment, the user's urine temperature and urinary hormone concentrations are automatically measured from the user's first morning urine each day, and her ovulation cycle is determined on the basis of the fluctuations in the measured urine temperature and urinary hormone concentrations. Therefore, the user can know her ovulation cycle with higher accuracy without having to take the burdensome measurement of her basal body temperature. In other words, the user can more easily manage her hormone balance and body temperature fluctuations using the present system, and can make use of this system for female health-care, birth control, and schedule management.

Moreover, according to the ovulation cycle monitor system 100 of the present embodiment, the user can know her current ovulation cycle as well as the dates of her next ovulation and onset of her next menstruation. On account of this, regardless of whether she wishes to become pregnant or not, it is convenient to use the system 100 when practicing birth control and planning activities, such as trips and intercourse. Especially for a woman who has an unpleasant symptom like dysmenorrheal or premenstrual syndrome peculiar to a certain phase of the ovulation cycle, the system 100 is more useful because she can plan her daily life in advance to avoid a hard schedule to relax during the certain phase by previously knowing the dates of her next ovulation and onset of menstruation of her next ovulation cycle.

The ovulation cycle calculating unit 114 predicts the ovulation cycle on the basis of the fluctuations in the secretion amounts of the urinary hormones, such as LH, FSH, estrogen, and progesterone, which fluctuate with the phases of the ovulation cycle. Thus, the ovulation cycle can be predicted with higher accuracy. Also, the unit 114 can provide information with a higher degree of reliability when the user plans her activity schedule.

In the present embodiment, the main unit 110 is provided for the main unit 210 mounted on the toilet fixture. However, via a radio communication network or short-range radio communication, the main unit 110 may be provided for a personal computer, server apparatus, personal digital assistant (PDA), or cellular phone that is set outside the toilet fixture and located inside or outside the housing. A specific explanation is given for a case where the main unit 110 is realized as a personal computer which is set inside the housing but outside the toilet fixture. In this case, the I/F unit 104 and the I/F unit 111 are used as short-range radio communication interfaces. Via this radio communication, data obtained through the measurement by the measuring unit 101 mounted on the toilet apparatus 200 and a control signal outputted from the ovulation cycle predicting unit 115 requesting to start measuring the urinary hormone concentrations are sent/received in real time. Here, the personal computer that serves as the main unit 110 and the toilet apparatus that serves as the measuring unit 101 are set at separate locations even though the locations are inside the same housing. For this reason, it is highly possible to cause a time lag between the timings of urine excretion and input of the identifying information corresponding to the measurement data. Against this backdrop, if there are no inputs from the user after a set period of time has passed after the identifying information list and prompting guidance were displayed, for example, the ovulation cycle calculating unit 114 categorizes the received result as the measurements belonging to a specific individual and stores them into the memory unit 117. Alternatively, when the main unit 110 is activated, such measurement data (for which the identifying information has not been inputted) may be listed together with the measurement date/time so as to accept the input of identifying information from the user who is the person being tested.

In a case where the main unit 110 is used as a personal computer, server apparatus, personal digital assistant (PDA), or cellular phone, each unit except for the information processing unit 113 can be realized by a corresponding function which is usually provided for the personal computer, personal digital assistant (PDA), or cellular phone that has a communication function. The information processing unit 113 can be realized by a program that is previously installed in the memory unit 117 and is executed by a CPU.

In the stated embodiment, the measuring unit 101 and the main unit 110 are connected via the cable. However, the connection may be established via infrared data communication instead of the cable. In this case, the I/F unit 104 and the I/F unit 111 are used as infrared data communication interfaces.

Moreover, in the stated embodiment, the explanation has been given for a case where the measuring unit 101 and the main unit 110 are mounted respectively on the toilet bowl and on the bathroom wall. However, the present invention is not limited to this case, and both the measuring unit 101 and the main unit 110 may be integrally mounted on the toilet seat or bowl that realizes the toilet apparatus.

As shown in FIG. 3, the urine temperature measuring unit 102 is realized by the infrared sensor 202 in the above embodiment. However, the urine temperature measuring unit 102 may measure the urine temperature using a thermistor provided for the urine receiver that collects the urine. In this case, another function may be provided so that the bathroom temperature will be measured before urination and the data of urine temperature given by the urine temperature measuring unit 102 will be corrected.

Also, the explanation is given for a case where the seat sensor 105 is a pressure sensor that detects a weight when the user sits on the toilet seat. However, the present invention is not limited to this, and the seat sensor 105 may be an infrared sensor or the like that detects the user's presence on the toilet seat by sensing the user's body temperature.

The communication unit 119 establishes communication via the radio phone communication network such as a cellular phone communication network. However, the network does not need to be wireless, and communication may be established via a usual public telephone line network. In this case, the antenna 120 is not required.

In the above embodiment, the hormone measuring unit 103 measures hormones, such as estrogen, progesterone, FSH, and LH, that fluctuate in the amount of secretion with the phases of the female menstrual cycle. However, not all of them have to be measured, and at least one of these hormones may be measured. Based on the measured hormone concentration and urine temperature, the user's ovulation cycle may be determined. In this case, it is preferable to measure one or more hormones that include LH. Also, as the hormones to be measured for predicting the ovulation cycle, LH, progesterone, estrogen, and FSH are given in the stated embodiment. The hormones subject to the measurement are not limited to these, and may be other hormones as long as they are measurable urine components and fluctuate with the phases of the ovulation cycle.

Moreover, as the hormone concentration measuring method employed by the hormone measuring unit 203, methods other than the one described in the embodiment may be used. Note that, supposing that more than one method are employed for measuring the hormone concentrations, the method of measuring the levels of FSH and LH is slightly different from the method of measuring the levels of estrogen and progesterone. More specifically, for measuring the levels of FSH and LH, the immunologic methods using antibodies that bind specifically to FSH and LH are frequently employed. The major examples are: enzyme immunoassay (EIA); chemiluminescence immunoassay; immunoagglutination method using latex particles that are bound to antibodies; and immunochromatography method using gold colloids or the like that are bound to antibodies. For measuring estrogen and progesterone, the immunologic methods using antibodies that bind specifically to estrogen and progesterone are frequently employed. The major examples are: enzyme immunoassay (EIA); chemiluminescence immunoassay; and fluorescence immunoassay. In addition to these, radioimmunoassay (RIA) using radioisotopes may be used when its safety in handling is assured in the future.

Although the seat sensor 204 is provided for the measuring unit 101 in the stated embodiment, the present invention is not limited to this and the seat sensor 204 does not need to be provided. In a case where the seat sensor 204 is not provided, each unit of both the toilet apparatus 200 and the main unit 210 may be set on standby by the user's manipulation of the operation panel 212 provided for the main unit 210. Moreover, in the stated embodiment, when the seat sensor 204 detects the user sitting on the toilet seat, the sitting detection signal is sent to each unit of both the toilet apparatus 200 and the main unit 210 so as to set them on standby. However, in a case where the urinary hormone concentrations are measured each morning, not a few days before/after the ovulation day as described in the embodiment, the seat sensor 204 may output the sitting detection signal only to the infrared sensor 202 and the hormone measuring unit 203, for example.

In the above embodiment, an e-mail notifying the user's partner of the onset of the user's fertile phase shows both one image selected by the user from the plurality of pre-stored images and character strings previously inputted by the user. However, this notification e-mail may be a predetermined one stored in the memory unit 117. Also, the e-mail may include only images or only character strings.

In the stated embodiment, each user previously sets a number as identifying information specifying the user, so that the user can input her identifying information by selecting her pre-registered number. However, the user identification method of the present invention is not limited to this. For example, a scale may be built in the toilet seat, and each user may previously store her weight into the ovulation cycle monitor system 100. Every time the user's weight is measured by the built-in scale when she sits on the toilet seat, the information processing unit 113 compares this measured weight with the weight that is pre-stored for the user. As the comparison result, if a difference between the measured weight and the pre-stored weight falls within a predetermined range (± 0.5kg, for instance), the measured weight is judged to agree with the pre-stored weight. Accordingly, the user can be identified on the basis of the pre-stored weight. In the case where two or more users have the similar weight, with a difference being about 1kg, it is preferable to use this method in combination with the stated method that uses a pre-stored number assigned for each user.

Moreover, besides the weight, the user may be identified by her height, for example. In this case, a sensor for measuring the user's height is set at the bathroom door, and the measured height may be compared with the height previously stored for the user. As is the case of the above weight method, if two or more users have the similar height, it is preferable to use this height method in combination with the method that uses a pre-stored number assigned for each user.

Furthermore, besides the methods based on the weight and height, the weight distribution over the toilet seat may be stored for each user. Every time a user sits on the toilet seat, the pressure distribution over the toilet seat may be measured for the user identification.

Alternatively, the user may be identified by her fingerprints or profile of the face. Moreover, a circuit may be provided for an accessory that a user wears, such as a wristwatch and a ring, or for a device that the user always carries, such as a cellular phone. With this circuit, the user's identifying information may be transmitted to the ovulation cycle monitor system 100 via wireless or cellular phone communication. Also, identifying information that is readable by radio waves may be written onto an electronic tag, such as an RFID tag or a wireless IC tag, and the ovulation cycle monitor system 100 may read the identifying information by emitting radio waves.

The main unit and the measuring unit of the ovulation cycle monitor system of the present invention are also useful as toilet fixtures that are systematically mounted in a bathroom. Moreover, the main unit and the measuring unit of the ovulation cycle monitor system of the present invention are also useful as a toilet seat or toilet bowl which is integrally provided with a bidet or the like. Furthermore, the main unit of the ovulation cycle monitor system of the present invention is useful as a personal computer, server apparatus, personal digital assistant (PDA), or cellular phone that has a communication function.

Although the present invention has been fully described by way of examples with reference to the accompanying drawings, it is to be noted that various changes and modifications will be apparent to those skilled in the art. Therefore, unless otherwise such changes and modifications depart from the scope of the present invention, they should be constructed as being included therein.

## Claims

1. An ovulation cycle monitor system that is provided for a toilet fixture, comprising:
a urine temperature measuring unit operable to measure a temperature of urine excreted from a female user;
a hormone concentration measuring unit operable to measure a concentration of a hormone that is contained in the excreted urine and fluctuates in a secretion amount with an ovulation cycle;
a memory unit operable to accumulate data of the measured urine temperature and data of the measured hormone concentration in association with a measurement day; and
a determining unit operable to determine the ovulation cycle of the female user by reference to the fluctuations in the urine temperature and in the hormone concentration accumulated in the memory unit.

2. The ovulation cycle monitor system according to Claim 1,
wherein the determining unit includes:
an ovulation cycle calculating unit operable to calculate an onset date and a length for each phase of the ovulation cycle by reference to the data of the measured urine temperature and the data of the measured hormone concentration accumulated in the memory unit; and
an ovulation cycle determining unit operable to determine future ovulation cycles of the female user on the basis of the onset date and the length calculated for each phase.

3. The ovulation cycle monitor system according to Claim 2,
wherein the ovulation cycle calculating unit calculates an average length for each phase by reference to the data of the measured urine temperature and the data of the measured hormone concentration accumulated over a plurality of ovulation cycles in the memory unit.

4. The ovulation cycle monitor system according to Claim 1,
wherein the hormone concentration measuring unit measures the concentration of at least one of estrogen, progesterone, follicle-stimulating hormone, and luteinizing hormone.

5. The ovulation cycle monitor system according to Claim 4,
wherein the determining unit determines, by reference to the data of the measured urine temperature and data of fluctuations in the measured concentration of the luteinizing hormone, an ovulation date on a basis that an ovulation day comes after a predetermined period of time following a time at which the urine temperature is within a predetermined low temperature range and the luteinizing hormone reaches a predetermined concentration range.

6. The ovulation cycle monitor system according to Claim 1, further comprising
an indicating unit operable to indicate the determined ovulation cycle to the female user,
wherein the indicating unit indicates at least one of a menstrual phase, an ovulation phase, a follicular phase, a luteal phase, and a fertile phase which make up the ovulation cycle of the female user.

7. The ovulation cycle monitor system according to Claim 6, further comprising:
a receiving unit operable to receive an input of a date of a menstruation onset from the female user so that each of the indicated phases is corrected; and
a correcting unit operable to correct an onset date for each phase of the determined ovulation cycle, on the basis of the inputted date of the menstruation onset.

8. The ovulation cycle monitor system according to Claim 1, further comprising:
an address registering unit operable to receive a registration input of an e-mail address from the female user and to store the registered e-mail address into the memory unit; and
a notifying unit operable to send an e-mail to the registered e-mail address to notify the onset of at least one of a menstrual phase, an ovulation phase, a follicular phase, a luteal phase, and a fertile phase which make up the determined ovulation cycle.

9. An ovulation cycle monitor method, comprising:
a step of measuring a temperature of urine excreted from a female user and a urinary hormone concentration; and
a step of determining an ovulation cycle of the female user that includes a menstrual phase, an ovulation phase, a follicular phase, a luteal phase, and a fertile phase, by reference to fluctuations in the measured urine temperature and in the measured urinary hormone concentration.

10. A program that is used for an ovulation cycle monitor system provided for a toilet fixture, causing a computer to function as:
a urine temperature measuring unit operable to measure a temperature of urine excreted from a female user;
a hormone concentration measuring unit operable to measure a concentration of a hormone that is contained in the excreted urine and fluctuates in a secretion amount with an ovulation cycle;
a memory unit operable to accumulate data of the measured urine temperature and data of the measured hormone concentration in association with a measurement day; and
a determining unit operable to determine the ovulation cycle of the female user by reference to the fluctuations in the urine temperature and in the hormone concentration accumulated in the memory unit.

11. A toilet apparatus that is provided for a toilet fixture, comprising:
a urine temperature measuring unit operable to measure a temperature of urine excreted from a female user;
a hormone concentration measuring unit operable to measure a concentration of a hormone that is contained in the excreted urine and fluctuates in a secretion amount with an ovulation cycle;
a memory unit operable to accumulate data of the measured urine temperature and data of the measured hormone concentration in association with a measurement day; and
a determining unit operable to determine the ovulation cycle of the female user by reference to the fluctuations in the urine temperature and in the hormone concentration accumulated in the memory unit.
